# FASCICULE DE BREVET EUROPEEN

(11) **EP 1 385 489 B1**
(45) Date de publication et mention de la délivrance du brevet: **11.01.2006**
(21) Numéro de dépôt: 02735534.6
(22) Date de dépôt: 03.05.2002
(51) Int. Cl.: A61K 9/50, A61K 9/26, A61K 38/55, A61K 31/401, A61P 9/12

(54) **GRANULES ENROBES A BASE D'INHIBITEUR DE L'ENZYME DE CONVERSION DE L'ANGIOTENSINE**
UMHÜLLTE PELLETS AUF BASIS EINES ACE-HEMMERS
COATED GRANULES BASED ON ANGIOTENSIN-CONVERTING ENZYME INHIBITOR

(30) Priorité: 09.05.2001 FR 0106120
(43) Date de publication de la demande: 04.02.2004
(73) Titulaire: ETHYPHARM, 78550 Houdan (FR)
(72) Inventeur: CHENEVIER, Philippe, Montréal, Quebec H2V 4K8 (CA)
(74) Mandataire: Touati, Catherine
(86) Numéro de dépôt international: PCT/FR2002/001535
(87) Numéro de publication internationale: WO 2002/089775

(56) Documents cités:
- EP-A- 0 288 732
- EP-A- 0 317 878
- WO-A-00/51593
- WO-A-01/19348
- WO-A-01/49272
- FR-A- 2 766 089

## Description

La présente invention concerne des granules enrobés à base d'inhibiteur de l'enzyme de conversion de l'angiotensine (IEC), ainsi que leur procédé de préparation et les comprimés orodispersibles comprenant lesdits granules enrobés.

Au sens de la présente invention, on entend par comprimés orodispersibles, un comprimé capable de se désagréger dans la bouche en moins de 60 secondes, de préférence en moins de 40 secondes au contact de la salive en formant une suspension aisée à avaler.

Les inhibiteurs de l'enzyme de conversion de l'angiotensine (IEC) sont utilisés en thérapeutique dans le traitement de l'hypertension artérielle essentielle ainsi qu'à la suite de l'infarctus du myocarde chez les patients stabilisés présentant des signes de dyefonctions ventriculaires gauches.

Les IEC les plus connus sont les suivants: Alacepril, Benazepril, Captopril, Ceronapril, Cilazapril, Delapril, Enalapril, Enalaprilat, Fosinopril, Imidapril, Lisinopril, Moveltopril, Perindopril, Quinapril, Ramipril, Spirapril, Temocapril et Tandolapril.

WO00/51593 envisage la possibilité de préparer des compositions orales se dissolvant dans la bouche en moins de 60 secondes contenant une mousse solide formée à partir de protéines et utilisant l'enalapril comme substance active.

Ces différents IEC sont disponibles sous forme de comprimés ou de gélules.

Par exemple, le Ramipril est disponible, en fonction des pays, sous la forme d'un comprimé ou d'une gélule, tous deux commercialisés par Aventis sous la marque Altace®.

Ces formes, de par leur simplicité d'utilisation, sont parfaitement adaptées au traitement ambulatoire. Cependant, certains patients et notamment les personnes âgées, connaissent des difficultés de déglutition telles qu'il leur est difficile et par conséquent désagréable d'ingérer des comprimés ou des gélules, même avec une prise associée de liquide.

Il est estimé que 50 % de la population a des difficultés pour avaler des comprimés ou des gélules. Ce problème a pour conséquence la non prise du médicament prescrit et ainsi une forte incidence sur l'efficacité du traitement (H. Seager, 1998, J. Pharm. Pharmacol. 50, 375-382).

Bien que la forme gélule permette d'ouvrir l'enveloppe externe (cupule de gélatine) et d'administrer le contenu dans une boisson ou un aliment, le goût très désagréable de ces IEC ne permet pas d'envisager ce mode d'administration de façon satisfaisante pour les patients.

Il n'existe pas à ce jour de composition pharmaceutique d'IEC permettant une administration aisée pour les patients ayant des problèmes de déglutition ou pour les personnes désirant prendre leur traitement sans une ingestion concomitante de liquide.

Des comprimés multiparticulaires à délitement rapide ont déjà été décrits par la Société demanderesse dans les brevets FR 99 02516, FR 97 09233, FR 98 14034, FR 92 08642 et FR 91 09245, mais aucun de ces brevets ne décrit des comprimés orodispersibles contenant un IEC.

Il est donc apparu souhaitable de remédier à cette situation et de développer un comprimé orodispersible renfermant un IEC et dont le goût et la palatabilité soient tout à fait acceptables, de manière à ce que l'administration de ce comprimé ne soit pas désagréable.

Et la Société Demanderesse a trouvé, que ces caractéristiques étaient obtenues avec un comprimé à base d'IEC sous forme de granules enrobés et d'un mélange d'excipients comprenant au moins un agent de désintégration, un agent soluble et un agent lubrifiant et éventuellement un agent gonflant, un agent perméabilisant, des édulcorants et des arômes.

En outre un tel comprimé permet d'obtenir des paramètres pharmacocinétiques équivalents à ceux qui peuvent être obtenus avec les comprimés ou gélules déjà existants.

Les comprimés conformes à l'invention présentent de manière surprenante, un goût agréable, malgré la désagrégation du comprimé dans la bouche et une libération du principe actif équivalente à celle des formes existantes.

Les comprimés conformes à l'invention comprennent des granules enrobés à base d'inhibiteur de l'enzyme de conversion de l'angiotensine, des isomères ou des sels pharmaceutiquement acceptables dudit inhibiteur.

La présente invention a également pour objet ces granules enrobés.

Selon un mode de réalisation avantageux des granules conformes à l'invention, l'inhibiteur de l'enzyme de conversion de l'angiotensine est choisi dans le groupe comprenant notamment le Bénazépril, le Captopril, le Cilazapril, l'Enalapril, le Sosinopril, le Lisinopril, le Périndopril, le Quinapril, le Ramipril, le Trandolapril, leurs isomères et leurs sels pharmaceutiquement acceptables.

Le masquage du goût de l'IEC est obtenu par enrobage, par un ou plusieurs polymères, des microcristaux granulés d'IEC. La répartition granulométrique et les propriétés mécaniques de ces granules permettent leur utilisation dans la fabrication de comprimés multiparticulaires à délitement rapide.

Les granules conformes à l'invention à base de microcristaux d'IEC, de ses isomères et de ses sels pharmaceutiquement acceptables, sont caractérisés par le fait qu'ils sont enrobés et qu'ils contiennent :
- des microcristaux d'IEC,
- un ou plusieurs agents liants,
- éventuellement un agent diluant et un agent antistatique.

La plupart des IEC sont disponibles sous forme de microcristaux de petite taille. Par exemple, le Ramipril est disponible dans le commerce sous forme de microcristaux dont la taille moyenne est inférieure à 100 µm. L'inconvénient d'une telle granulométrie réside dans le fait que l'enrobage par les méthodes consistant à pulvériser une solution ou une suspension d'enrobage sur ces microcristaux dans un appareil à lit d'air fluidisé est difficile et long.

Pour remédier à cet inconvénient, conformément à l'invention, les microcristaux d'IEC sont granulés et enrobés de façon à conduire à des particules qui présentent une granulométrie telle qu'au moins 80 % des particules ont une taille comprise entre 100 et 500 µm, et moins de 15 % des particules ont une taille inférieure à 100 µm.

Dans les granules enrobés conformes à l'invention, l'IEC conserve son intégrité physico-chimique, la granulation et l'enrobage ne modifiant en rien les propriétés intrinsèques du principe actif.

Selon la présente invention, les granules comprennent un agent liant qui permet de lier les microcristaux d'IEC et les autres constituants éventuels, afin de donner des granules dont la taille rendra plus aisée l'opération d'enrobage.

Cet agent liant peut être choisi dans le groupe comprenant notamment des polymères cellulosiques, des povidones, des polyvinylalcools et des polymères acryliques.

Parmi les polymères cellulosiques, on choisira avantageusement l'éthycellulose, l'hydroxypropylcellulose (HPC) et l'hydroxypropylméthylcellulose (HPMC), seuls ou en mélange.

Parmi les polymères acryliques, on choisira avantageusement le copolymère ammonio-métacrylate (Eudragit® RL et RS), le polyacrylate (Eudragit® NE) et le polymétacrylate (Eudragit® E), Eudragit® étant une marque déposée. Ces polymères sont utilisés seuls ou en mélange.

L'agent liant est présent dans des proportions pouvant aller jusqu'à 15 % en poids, de préférence jusqu'à 10 % en poids par rapport au poids des granules non enrobés.

De façon avantageuse, afin de favoriser la granulation de l'IEC, on utilise un agent diluant. Cet agent diluant permet également d'augmenter la proportion de granules enrobés dans les comprimés et de limiter ainsi le risque d'hétérogénéité lié aux faibles dosages des IEC.

Cet agent diluant peut être choisi dans le groupe comprenant notamment les dérivés cellulosiques et préférentiellement la cellulose microcristalline, les amidons, le lactose, les polyols et préférentiellement le mannitol.

Cet agent diluant est présent dans des proportions pouvant aller jusqu'à 85 % en poids, de préférence jusqu'à 50 % en poids par rapport au poids des granules non enrobés.

De façon avantageuse, afin de faciliter la fluidisation de la matière lors de l'utilisation d'un lit d'air fluidisé pour la granulation, on peut utiliser un agent ayant des propriétés antistatiques.

Cet agent antistatique peut être choisi dans le groupe comprenant notamment la silice colloïdale, notamment celle commercialisée sous la marque Aerosil®, et préférentiellement la silice précipitée, notamment celle commercialisée sous le nom Syloïd® FP244, le talc micronisé ou non, et leurs mélanges.

Cet agent antistatique est présent dans des proportions pouvant aller jusqu'à 10% en poids, de préférence jusqu'à 3% en poids par rapport au poids des granules non enrobés.

Les granules obtenus après la granulation sont enrobés par pulvérisation d'une composition d'enrobage notamment sous forme de solution ou de suspension de polymère ce qui permettra d'obtenir un masquage du goût de l'IEC.

La composition d'enrobage est choisie en fonction des caractéristiques physico-chimiques de l'IEC et est constituée d'au moins un polymère d'enrobage et d'éventuellement un agent antistatique, de plastifiants et d'agents solubles, notamment de polyols.

Le polymère d'enrobage est choisi de façon avantageuse dans le groupe comprenant les polymères cellulosiques, les polymères acryliques, et leurs mélanges.

Parmi les polymères cellulosiques, on choisira avantageusement l'éthycellulose, l'hydroxypropylcellulose (HPC) et l'hydroxypropylméthylcellulose (HPMC), seuls ou en mélange.

Parmi les polymères acryliques, on choisira avantageusement le copolymère amonio-métacrylate (Eudragit® RL et RS), le polyacrylate (Eudragit® NE) et le polymétacrylate (Eudragit® E), Eudragit® étant une marque déposée par RÖHM.

Le polymère d'enrobage est présent dans des proportions pouvant aller jusqu'à 30 % en poids, de préférence jusqu'à 15 % en poids par rapport au poids des granules enrobés.

L'agent antistatique peut être choisi dans le groupe comprenant notamment la silice colloidale, notamment celle commercialisée sous la marque Aerosil®, et préférentiellement la silice précipitée, notamment celle commercialisée sous la marque Syloïd® FP244, le talc micronisé ou non, et leurs mélanges.

Cet agent est présent dans des proportions pouvant aller jusqu'à 10 % en poids, de préférence jusqu'à 5 % en poids par rapport au polymère d'enrobage.

Selon un mode de réalisation avantageux de l'invention,
un même polymère constitue l'agent liant du granule et le polymère d'enrobage.

Selon un mode de réalisation avantageux, les granules enrobés de la présente invention comprennent:
- de 10 à 95 % de microcristaux d'IEC,
- de 0 à 85 % d'un agent diluant,
- de 0 à 10 % d'un agent antistatique,
- de 5 à 50 % d'un polymère d'enrobage,

les pourcentages étant exprimés en poids par rapport au poids du granule enrobé.

Des concentrations en agent diluant supérieures à 85 %, ne sont pas avantageuses du point de vue de la facilitation de la granulation et entraînent une dilution importante de l'IEC, d'où l'obtention de comprimé de taille importante.

Pour une concentration en polymère d'enrobage inférieure à 5%, l'enrobage n'est pas suffisant pour permettre un bon masquage du goût. Pour une concentration supérieure à 50 %, la libération de l'IEC est trop ralentie.

Des concentrations en agent antistatique supérieures à 10 %, n'entraînent pas d'amélioration supplémentaire de la fluidisation des particules.

Selon un autre mode de réalisation avantageux, les granules enrobés de l'invention comprennent:
- de 10 à 95 % de microcristaux d'IEC,
- de 0 à 85 % de mannitol,
- de 0 à 10 % de silice colloidale, et
- de 5 à 50 % d'éthylcellulose,

les pourcentages étant exprimés en poids par rapport au poids du granule enrobé.

L'invention porte également sur le procédé de préparation des granules enrobés d'IEC.

Le procédé conforme à l'invention comprend successivement les étapes:
- de mélange à sec des microcristaux d'IEC avec l'agent diluant et éventuellement un agent antistatique,
- de granulation du mélange obtenu à l'étape précédente par pulvérisation d'une solution ou suspension de l'agent liant,
- d'enrobage des granules ainsi obtenus par pulvérisation d'une suspension de la composition d'enrobage,
- de séchage des granules enrobés ainsi obtenus.

Selon ce procédé, les étapes de mélange, de granulation et d'enrobage peuvent être réalisées au sein d'appareils différents ou du même appareil et en présence, pour chaque étape, d'un mélange d'excipients de nature identique ou différente.

Dans un premier mode de réalisation du procédé de granulation conforme à l'invention, les étapes de mélange à sec initial, de granulation, d'enrobage et de séchage sont effectuées en lit d'air fluidisé.

Dans ce cas, le mélange initial de poudre de principe actif, d'agent diluant et éventuellement d'agent antistatique est tout d'abord fluidisé, avant d'être granulé par pulvérisation sur ladite poudre d'une solution ou suspension d'excipients comprenant au moins un agent liant, les grains obtenus sont ensuite enrobés par pulvérisation de la suspension de composition d'enrobage, les granulés enrobés formés sont séchés, l'ensemble de ces étapes étant effectué dans un lit fluidisé.

Selon un mode de réalisation avantageux, le mélange d'excipients mis en oeuvre lors de l'étape de granulation et la suspension d'enrobage mise en oeuvre lors de l'étape d'enrobage forment un seul mélange. Dans ce cas, l'étape de granulation se distingue de l'étape d'enrobage en faisant varier différents paramètres, tels que le débit de pulvérisation du mélange d'excipients et la pression d'atomisation dudit mélange. Ainsi, une partie seulement du mélange d'excipients est mise en oeuvre lors de l'étape de granulation tandis que l'autre partie est mise en oeuvre lors de l'étape d'enrobage.

Le débit de pulvérisation de la suspension d'excipients est plus élevé lors de l'étape de granulation, que lors de l'étape d'enrobage, tandis que la pression d'atomisation de la suspension d'excipients est moins élevée lors de l'étape de granulation que lors de l'étape d'enrobage.

En pratique, à l'échelle du laboratoire au sein d'un appareil à lit d'air fluidisé par exemple du type GLATT GPCG3, lors de l'étape de granulation, le débit de pulvérisation du mélange d'excipients est compris entre 15 et 30 grammes/minute, et la pression d'atomisation est comprise entre 1 et 2,5 bars.

Lors de l'étape d'enrobage, le débit de pulvérisation de la suspension d'enrobage est compris entre 10 et 25 grammes/minute, tandis que la pression d'atomisation est comprise entre 1,5 et 3 bars.

Selon un mode de réalisation avantageux, de 10 à 30 % du mélange d'excipients sont pulvérisés pendant l'étape de granulation, le complément à 100 % étant pulvérisé pendant l'étape d'enrobage.

Selon ce procédé avantageux, après avoir mélangé à sec le principe actif, l'agent diluant et avantageusement l'agent antistatique, on pulvérise sur le lit fluidisé une suspension d'excipients comprenant le ou les polymères d'enrobage, l'agent antistatique en faisant varier le débit de pulvérisation et la pression d'atomisation de ladite suspension, de façon à obtenir tout d'abord une granulation puis ensuite un enrobage des grains formés.

Les granules enrobés à base d'IEC ainsi préparés sont tout particulièrement destinés à entrer dans la constitution de comprimés à délitement rapide qui sont également un objet de l'invention.

Les comprimés selon l'invention sont destinés à se désagréger dans la bouche au contact de la salive en moins de 60 secondes, de préférence en moins de 40 secondes, en formant une suspension aisée à avaler, et ils sont à base de granules enrobés d'IEC et d'un mélange d'excipients comprenant au moins un agent de désintégration, un agent soluble diluant, un agent lubrifiant et éventuellement un agent gonflant, un agent perméabilisant, des édulcorants et des arômes.

Le temps de désagrégation correspond à la durée qui sépare, d'une part, le moment de la mise en place du comprimé dans la bouche au contact de la salive et, d'autre part, le moment de la déglutition de la suspension résultant de la désagrégation sans mastication du comprimé au contact de la salive.

Selon un mode de réalisation avantageux, les comprimés conformes à l'invention sont à base de granules enrobés d'IEC, lesdits granules présentant des caractéristiques de compression intrinsèques et d'un mélange d'excipients, la proportion de mélange d'excipients par rapport aux granules enrobés étant de 0,4 à 10, de préférence de 1 à 10 et plus préférentiellement encore de 1 à 4 parties en poids, le mélange d'excipients comprenant :
- un agent de désintégration,
- un agent soluble diluant à propriétés liantes,
- un lubrifiant,
- un agent perméabilisant, et
- éventuellement des édulcorants, des arômes et des colorants.

L'agent de désintégration est choisi dans le groupe comprenant notamment la carboxyméthylcellulose sodique réticulée désignée dans le métier par le terme croscarmellose, la crospovidone et leurs mélanges.

L'agent soluble diluant à propriétés liantes, constitué par un polyol de moins de 13 atomes de carbone se présentant soit sous forme de produit directement compressible dont le diamètre moyen des particules est de 100 à 500 µm, soit sous la forme d'une poudre dont le diamètre moyen des particules est inférieur à 100 µm, ce polyol étant de préférence choisi dans le groupe comprenant le mannitol, le xylitol, le sorbitol et le maltitol, étant entendu que le sorbitol ne peut pas être utilisé seul et que, dans le cas où l'agent soluble diluant à propriétés liantes serait unique, il est utilisé sous la forme du produit directement compressible alors que, dans le cas où il y aurait au moins deux agents solubles diluants à propriétés liantes, l'un est présent sous la forme directement compressible et l'autre sous la forme poudre, le polyol pouvant être le même, les proportions de polyol directement compressible et de polyol poudre étant de 99/1 à 20/80, de préférence de 80/20 à 20/80.

Les proportions respectives d'agent de désintégration et d'agent soluble retenues pour la constitution de l'excipient sont, par rapport à la masse du comprimé, de 1 à 15 % , de préférence de 2 à 7% en poids pour le premier et de 30 à 90 %, de préférence de 40 à 70% en poids pour le second.

Le lubrifiant est choisi dans le groupe comprenant notamment le stéarate de magnésium, l'acide stéarique, le stéarylfumarate de sodium, le polyoxyethylèneglycol micronisé (Macrogol 6000 micronisé), la leucine, le benzoate de sodium et leurs mélanges.

La quantité d'agent lubrifiant est de 0,2 à 20 pour mille (poids de l'agent lubrifiant/poids total du comprimé), de préférence de 5 à 10 pour mille.

L'agent de lubrification peut être réparti au sein de l'excipient ou selon un mode de réalisation avantageux, la totalité de l'agent lubrifiant peut être répartie à la surface du comprimé.

Comme agent perméabilisant on utilise un composé choisi dans le groupe comprenant notamment des silices ayant une grande affinité pour les solvants aqueux, telles que la silice précipitée plus connue sous le nom de marque Syloid®, les maltodextrines, les β-cyclodextrines et leurs mélanges.
L'agent perméabilisant permet la création d'un réseau hydrophile qui facilite la pénétration de la salive et contribue ainsi à une meilleure désagrégation du comprimé.

La proportion d'agent perméabilisant par rapport à la masse du comprimé est de 0,5 % à 5% en poids.

L'édulcorant peut être choisi dans le groupe comprenant notamment l'aspartam, l'acésulfame de potassium, le saccharinate de sodium, la néohespéridine dihydrochalcone, le sucralose, le monoammonium glycyrrhizinate, et leurs mélanges.

Les arômes et colorants sont ceux utilisés habituellement en pharmacie pour la préparation de comprimés.

L'invention sera mieux comprise au moyen du complément de description qui suit et qui se réfère à des exemples avantageux de réalisation des comprimés d'IEC à délitement rapide conformes à l'invention. Il va de soi, toutefois, que ces exemples sont donnés uniquement à titre d'illustrations et de modes de réalisation avantageux de l'invention et n'en constituent nullement une limitation.

### Exemple 1 : Préparation de granules enrobés à base de Ramipril.

Les granules sont fabriqués avantageusement en utilisant un granulateur à lit d'air fluidisé, selon le procédé suivant.

On prépare tout d'abord une solution de polymère en solubilisant 109 g d'éthycellulose dans 2050 g d'alcool isopropylique.

On fluidise ensuite un mélange homogène de poudres constitué de 500 grammes de Ramipril, de 500 grammes de mannitol et de 53 grammes de silice dans un lit d'air fluidisé.

La granulométrie des différentes poudres est inférieure à 100 µm.

On réalise la granulation par pulvérisation, sur le lit fluidisé du mélange homogène de poudres, d'une première fraction d'environ 15 à 25 % de la totalité de la solution de polymère pendant environ 20 minutes à un débit de pulvérisation d'environ 20 à 25 grammes/minutes et sous une pression d'atomisation de la solution de 2 bars.

On obtient ainsi un lit fluidisé de granules dont la granulométrie est majoritairement comprise entre 100 et 500 µm.

On procède ensuite à l'enrobage des granules en pulvérisant sur le lit fluidisé de granules la fraction restante de la solution de polymère, la durée de la pulvérisation étant d'environ 2 heures, le débit de pulvérisation d'environ 15 à 20 grammes/minutes et la pression d'atomisation de la solution de 2,5 bars.

On obtient ainsi un mélange homogène de granules enrobés.

Le mélange homogène de granules enrobés a une composition statistiquement constante.

Ainsi, il est possible de déterminer la teneur en principe actif d'une masse donnée de ce mélange homogène de granules enrobés, et, inversement, il est possible pour une quantité de principe actif souhaitée de déterminer la quantité correspondante de mélange homogène de granules enrobés.

Par exemple, pour avoir 10mg de Ramipril, il faudra prendre 24,62mg du mélange homogène de granules enrobés. La composition de ces 24,62mg est indiquée dans le tableau I ci-dessous:

**Tableau I**

| | | |
|---|---|---|
| Principe actif | Ramipril | 10 mg |
| Agent diluant | Mannitol | 10 mg |
| Agent liant/Agent d'enrobage | Ethycellulose | 3,12 mg |
| Agent antistatique | Silice précipitée | 1,5 mg |
| | TOTAL | 24,62 mg |

### Exemple 2 : Préparation de comprimés multiparticulaires à délitement rapide contenant 10 mg de Ramipril

On prépare des comprimés contenant 10mg de Ramipril dont la composition résulte du tableau II donné ci-après.

Des quantités de chacun des excipients identifiés dans le tableau II ci-dessous, pris en les proportions respectives correspondant avec les proportions résultant dudit tableau sont tamisés en vrac sur un tamis présentant une ouverture de maille d'environ 800 µm.

Puis, dans un mélangeur à sec on mélange de façon homogène une quantité de granules enrobés correspondant à la proportion de granules qui résulte du tableau II avec le mélange tamisé d'excipients.

**Tableau II**

| **Composition du comprimé contenant 10 mg de Ramipril** | | |
|---|---|---|
| Principe actif | Granules enrobés de l'exemple 1 | 24,62 mg équivalent à 10 mg de Ramipril |
| Agent de compression | Mannitol | 159 mg |
| Agent désintégrant | Crospovidone | 22 mg |
| Agent perméabilisant | Silice précipitée | 2,2 mg |
| Agent édulcorant | Aspartam | 4,4 mg |
| Aromatisant | Arôme orange | 2,2 mg |
| Agent lubrifiant | Stéarate de magnésium | 2,2 mg |

Le mélange obtenu est ensuite réparti et mis en forme sur une comprimeuse rotative équipée de matrices et de poinçons de diamètre 8 mm.

La dureté des comprimés obtenus est de 30 N environ.

Le temps de désagrégation dans la bouche des comprimés ainsi obtenus est inférieur à 20 secondes.

L'abrasion constitue une propriété importante des comprimés conformes à l'invention. Celle-ci est mesurée selon le procédé décrit dans la pharmacopée française (Xème édition, "V.5.1-friabilité des comprimés", Janvier 1993) à l'aide d'un appareil à ailettes et a été trouvée inférieure à 1 %.

Le goût désagréable du Ramipril n'est pas détecté pendant le temps de séjour du comprimé dans la bouche, c'est à dire entre le moment de son administration et le moment de la déglutition.

La cinétique de dissolution est déterminée selon la Pharmacopée Européenne 3^{ème} édition, dans un appareil de type II équipé de pales rotatives

Pour cela on introduit dans cet appareil, 1 comprimé dans un volume de dissolution de 500 ml en milieu HCl 0,1 N, et on agite à une vitesse de 75 tours par minute. On mesure à intervalles réguliers la concentration en Ramipril dissous dans le milieu.

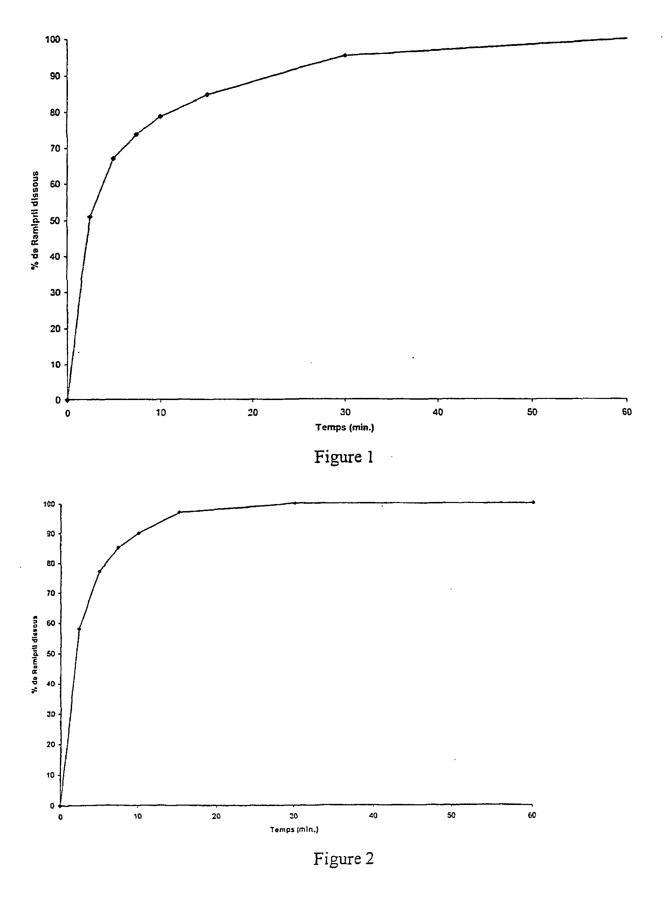

Les résultats obtenus sont reportés sur la figure 1 sous forme d'une courbe C₁ qui illustre la dissolution de la substance active en fonction du temps.

Comme le montre cette courbe C₁, les comprimés multiparticulaires à délitement rapide de Ramipril permettent d'obtenir une libération non retardée du Ramipril.

### Exemple 3 : Préparation de comprimés multiparticulaires à délitement rapide contenant 7,5 mg de Ramipril

On prépare des comprimés contenant 7,5 mg de Ramipril dont la composition résulte du tableau III ci-après:

**Tableau III**

| **Composition de comprimés de 7,5 mg de Ramipril** | | |
|---|---|---|
| Principe actif | Granules enrobés de l'exemple 1 | 18,53 mg équivalent à 7,5 mg de Ramipril |
| Agent de compression | Mannitol | 119 mg |
| Agent désintégrant | Crospovidone | 16,2 mg |
| Agent perméabilisant | Silice précipitée | 1,6 mg |
| Agent édulcorant | Aspartam | 3,2 mg |
| Aromatisant | Arôme Menthe | 1,6 mg |
| Agent lubrifiant | Stéarate de magnésium | 1,6 mg |

Pour la préparation de ces comprimés on procède comme dans l'exemple 2 en utilisant les excipients et le principe actif dans les proportions relatives résultant du tableau III ci-dessus.

Le poids des comprimés est de 162 mg.

La dureté des comprimés obtenus est de 30 N environ.

Le temps de désagrégation dans la bouche des comprimés ainsi obtenus est inférieur à 20 secondes.

L'abrasion mesurée comme dans l'exemple 2, a été trouvée comme étant inférieure à 1 %.

Le goût désagréable du Ramipril n'est pas détecté pendant le temps de séjour du comprimé dans la bouche, c'est à dire entre le moment de son administration du comprimé et le moment de la déglutition.

### Exemple 4 : Préparation de granules enrobés de Ramipril.

Les granules sont fabriqués selon le procédé suivant.

On prépare tout d'abord une solution de polymère en solubilisant l'Eudragit® E100 dans de l'alcool isopropylique.

On fluidise ensuite un mélange homogène de poudres constitué de 500g de Ramipril, 750g de mannitol et 53g de silice précipitée dans un lit d'air fluidisé.

La granulométrie des différentes poudres est inférieure à 100 µm.

Avantageusement la granulation du mélange de poudres est réalisée dans un granulateur à lit d'air fluidisé.

On réalise la granulation par pulvérisation sur le lit fluidisé du mélange homogène de poudres, d'une première fraction d'environ 15 à 25 % de la solution de polymère pendant environ 30 minutes à un débit de pulvérisation d'environ 20 à 25 grammes/minutes et une pression d'atomisation de la solution de 1,5 bars.

On obtient ainsi un lit fluidisé de granules dont la granulométrie est majoritairement comprise entre 100 et 500 µm. On procède ensuite à l'enrobage des granules en pulvérisant la fraction restante de la solution de polymère sur le lit fluidise de granules, la durée de pulvérisation étant d'environ 2 heures, le débit de pulvérisation d'environ 15 à 20 grammes/minutes et la pression d'atomisation de la solution de 2 bars.

On obtient alors un mélange de granules enrobés.

Le mélange homogène de granules enrobés a une composition statistiquement constante.

Ainsi, il est possible de déterminer la teneur en principe actif d'une masse donnée de ce mélange homogène de granules enrobés, et, inversement, il est possible pour une quantité de principe actif souhaitée de déterminer la quantité correspondante de mélange homogène de granules enrobés.

Par exemple, pour avoir 10 mg de Ramipril, il faudra prendre 31,5 mg du mélange homogène de granules enrobés. La composition de ces 31,5 mg est indiquée dans le tableau IV ci-dessous.

**Tableau IV**

| | | |
|---|---|---|
| Principe actif | Ramipril | 10 mg |
| Agent diluant | Mannitol | 15 mg |
| Agent liant/Agent d'enrobage | Eudragit® E 100 | 5 mg |
| Agent antistatique | Silice précipitée | 1,5 mg |
| | TOTAL | 31,5 mg |

### Exemple 5 : Préparation de comprimés multiparticulaires à délitement rapide contenant 10 mg de Ramipril

On prépare des comprimés contenant 10mg de Ramipril dont la composition résulte du tableau V ci-après :

**Tableau V**

| **Composition d'un comprimé contenant 10 mg de Ramipril** | | |
|---|---|---|
| Principe actif | Granules enrobés de l'exemple 4 | 31, 5 mg équivalent à 10 mg de Ramipril |
| Agent de compression | Mannitol | 231 mg |
| Agent désintégrant | Crospovidone | 25 mg |
| Agent perméabilisant | Silice précipitée | 1,5 mg |
| Agent édulcorant | Aspartam | 5 mg |
| Aromatisant | Arôme citron | 3 mg |
| Agent lubrifiant | Stéarate de magnésium | 3mg |

Pour la préparation de ces comprimés on procède comme dans l'exemple 2 en utilisant les excipients et le principe actif dans les proportions relatives résultant du tableau V ci-dessus.

Le poids des comprimés est de 300 mg.

La dureté des comprimés obtenus est de 35 N environ.

Le temps de désagrégation dans la bouche des comprimés ainsi obtenus est inférieur à 25 secondes.

L'abrasion, mesurée comme dans l'exemple 2, a été trouvée comme étant inférieure à 1 %.

Le goût désagréable du Ramipril n'est pas détecté pendant le temps de séjour du comprimé dans la bouche, c'est à dire entre le moment de son administration et là déglutition.

La cinétique de dissolution est réalisée selon la Pharmacopée Européenne 3^{ème} édition, dans un appareil de type II équipé de pales rotatives.

Pour cela on introduit dans cet appareil 1 comprimé dans un volume de dissolution de 500ml en milieu HCl 0,1 N, et on agite à une vitesse de 75 tours par minute. On mesure à intervalles réguliers la concentration en Ramipril dissous dans le milieu.

Les résultats obtenus sont reportés sur la figure 2 sous forme d'une courbe C₂ qui illustre la dissolution de la substance active en fonction du temps.

Comme le montre cette courbe C₂, les comprimés multiparticulaires à délitement rapide de Ramipril permettent d'obtenir une libération non retardée du Ramipril.

### Exemple 6 : Préparation de comprimés multiparticulaires à délitement rapide contenant 5 mg de Ramipril

Les granules de Ramipril décrits dans l'exemple 4 sont formulés en comprimés multiparticulaires à délitement rapide contenant 5 mg de Ramipril dont la composition est donnée dans le tableau VI ci-après:

**Tableau VI**

| **Composition d'un comprimé contenant 5 mg de Ramipril** | | |
|---|---|---|
| Principe actif | Granules enrobés de l'exemple 4 | 15,75 mg équivalent à 5 mg de Ramipril |
| Agent de compression | Mannitol | 115 mg |
| Agent désintégrant | Crospovidone | 12 mg |
| Agent perméabilisant | Silice précipitée | 0,7 mg |
| Agent édulcorant | Aspartam | 3 mg |
| Aromatisant | Arôme Menthe | 1,5 mg |
| Agent lubrifiant | Stéarate de magnésium | 1 mg |

On fabrique les comprimés selon le procédé de l'exemple 2 en utilisant les différents excipients et le principe actif dans les proportions données dans le tableau VI.

Le poids des comprimés est de 150 mg.

La dureté des comprimés obtenus est de 35 N environ.

Le temps de désagrégation dans la bouche des comprimés ainsi obtenus est inférieur à 20 secondes.

L'abrasion, mesurée comme dans l'exemple 2, a été trouvée comme étant inférieure à 1 %.

Le goût du Ramipril n'est pas détecté pendant le temps de séjour du comprimé dans la bouche, c'est à dire entre le moment de son administration et le moment de la déglutition.

### Exemple 7 : Préparation de granules enrobés de Captopril.

Les granules sont fabriqués selon le procédé suivant.

On prépare tout d'abord une solution de polymère en solubilisant l'Eudragit® E100 dans de l'alcool isopropylique.

on fluidise ensuite un mélange homogène de poudres constitué de 750g de Captopril, 250g de mannitol et 53g de silice précipitée dans un lit d'air fluidisé.

On réalise la granulation par pulvérisation sur le lit fluidisé du mélange homogène de poudres, d'une première fraction d'environ 15 à 25 % de la solution de polymère pendant environ 30 minutes à un débit de pulvérisation d'environ 20 à 25 grammes/minutes et une pression d'atomisation de la solution de 1,5 bars.

On obtient ainsi un lit fluidisé de granules dont la granulométrie est majoritairement comprise entre 100 et 500 µm

On procède ensuite à l'enrobage des granules en pulvérisant la fraction restante de la solution de polymère sur le lit fluidisé de granules, la durée de pulvérisation étant d'environ 2 heures, le débit de pulvérisation d'environ 15 à 20 grammes/minutes et la pression d'atomisation de la solution de 2 bars.

On obtient alors un mélange de granules enrobés.

Le mélange homogène de granules enrobés a une composition statistiquement constante.

Ainsi, il est possible de déterminer la teneur en principe actif d'une masse donnée de ce mélange homogène de granules enrobés, et, inversement, il est possible pour une quantité de principe actif souhaitée de déterminer la quantité correspondante de mélange homogène de granules enrobés.

Par exemple, pour avoir 50 mg de Captopril, il faudra prendre 76,9 mg du mélange homogène de granules enrobés. La composition de ces 76,9 mg est indiquée dans le tableau VII ci-dessous.

**Tableau VII**

| | | |
|---|---|---|
| Principe actif | Captopril | 50 mg |
| Agent diluant | Mannitol | 16,7 mg |
| Agent liant/Agent d'enrobage | Eudragit® E 100 | 6,7 mg |
| Agent antistatique | Silice précipitée | 3,5 mg |
| | TOTAL | 76,9mg |

### Exemple 8 : Préparation de comprimés multiparticulaires à délitement rapide contenant 50 mg de Captopril

On prépare des comprimés contenant 50mg de Captopril dont la composition résulte du tableau VIII ci-après :

**Tableau VIII**

| **Composition d'un comprimé contenant 50 mg de Captopril** | | |
|---|---|---|
| Principe actif | Granules enrobés de l'exemple 7 | 76,9 mg équivalent à 50 mg de Captopril |
| Agent de compression | Mannitol | 112 mg |
| Agent désintégrant | Crospovidone | 22 mg |
| Agent perméabilisant | Silice précipitée | 4 mg |
| Agent édulcorant | Aspartam | 5 mg |
| Aromatisant | Arôme citron | 2 mg |
| Agent lubrifiant | Stéarate de magnésium | 2 mg |

Pour la préparation de ces comprimés on procède comme dans l'exemple 2 en utilisant les excipients et le principe actif dans les proportions relatives résultant du tableau VIII ci-dessus.

Le poids des comprimés est de 224 mg.

La dureté des comprimés obtenus est de 40 N environ.

Le temps de désagrégation dans la bouche des comprimés ainsi obtenus est inférieur à 30 secondes.

L'abrasion, mesurée comme dans l'exemple 2, a été trouvée comme étant inférieure à 1 %.

Le goût désagréable du Captopril n'est pas détecté pendant le temps de séjour du comprimé dans la bouche, c'est à dire entre le moment de son administration et la déglutition.

### Exemple 9 : Préparation de granules enrobés d'Enalapril,

Les granules sont fabriqués selon le procédé suivant.

On prépare tout d'abord une solution de polymère en solubilisant l'éthylcellulose dans de l'alcool isopropylique.

On fluidise ensuite un mélange homogène de poudres constitué de 500g d'Enalapril, 500g de mannitol et 50g de silice précipitée dans un lit d'air fluidisé.

On réalise la granulation par pulvérisation sur le lit fluidisé du mélange homogène de poudres, d'une première fraction d'environ 15 à 25 % de la solution de polymère pendant environ 30 minutes à un débit de pulvérisation d'environ 20 à 25 grammes/minutes et une pression d'atomisation de la solution de 2 bars.

On obtient ainsi un lit fluidisé de granules dont la granulométrie est majoritairement comprise entre 100 et 500 µm

On procède ensuite à l'enrobage des granules en pulvérisant la fraction restante de la solution de polymère sur le lit fluidisé de granules, la durée de pulvérisation étant d'environ 2 heures, le débit de pulvérisation d'environ 15 à 20 grammes/minutes et la pression d'atomisation de la solution de 2.5 bars.

On obtient alors un mélange de granules enrobés.

Le mélange homogène de granules enrobés a une composition statistiquement constante.

Ainsi, il est possible de déterminer la teneur en principe actif d'une masse donnée de ce mélange homogène de granules enrobés, et, inversement, il est possible pour une quantité de principe actif souhaitée de déterminer la quantité correspondante de mélange homogène de granules enrobés.

Par exemple, pour avoir 10 mg d'Enalapril, il faudra prendre 23 mg du mélange homogène de granules enrobés. La composition de ces 23 mg est indiquée dans le tableau IX ci-dessous.

**Tableau IX**

| | | |
|---|---|---|
| Principe actif | Enalapril | 10 mg |
| Agent diluant | Mannitol | 10 mg |
| Agent liant/Agent d'enrobage | Ethylcellulose | 2 mg |
| Agent antistatique | Silice précipitée | 1 mg |
| | TOTAL | 23 mg |

### Exemple 10 : Préparation de comprimés multiparticulaires à délitement rapide contenant 10 mg d'Enalapril

On prépare des comprimés contenant 10mg d'Enalapril dont la composition résulte du tableau X ci-après :

**Tableau X**

| **Composition d'un comprimé contenant 10 mg de Enalapril** | | |
|---|---|---|
| Principe actif | Granules enrobés de l'exemple 9 | 23 mg équivalent à 10 mg d'Enalapril |
| Agent de compression | Mannitol | 120 mg |
| Agent désintégrant | Crospovidone | 10 mg |
| Agent perméabilisant | Silice précipitée | 1,5 mg |
| Agent édulcorant | Aspartam | 3 mg |
| Aromatisant | Arôme Menthe | 2 mg |
| Agent lubrifiant | Stéarate de magnésium | 1,5 mg |

Pour la préparation de ces comprimés on procède comme dans l'exemple 2 en utilisant les excipients et le principe actif dans les proportions relatives résultant du tableau X ci-dessus.

Le poids des comprimés est de 161 mg.

La dureté des comprimés obtenus est de 30 N environ.

Le temps de désagrégation dans la bouche des comprimés ainsi obtenus est inférieur à 25 secondes.

L'abrasion, mesurée comme dans l'exemple 2, a été trouvée comme étant inférieure à 1 %.

Le goût désagréable de l'Enalapril n'est pas détecté pendant le temps de séjour du comprimé dans la bouche, c'est à dire entre le moment de son administration et la déglutition.

### Exemple 11 : Préparation de granules enrobés de Lisinopril.

Les granules sont fabriqués selon le procédé suivant.

On prépare tout d'abord une solution de polymère en solubilisant l'Eudragit® E100 dans de l'alcool isopropylique.

On fluidise ensuite un mélange homogène de poudres constitué de 500g de Lisinopril, 500g de mannitol et 50g de silice précipitée dans un lit d'air fluidisé.

On réalise la granulation par pulvérisation sur le lit fluidisé du mélange homogène de poudres, d'une première fraction d'environ 15 à 25 % de la solution de polymère pendant environ 30 minutes à un débit de pulvérisation d'environ 20 à 25 grammes/minutes et une pression d'atomisation de la solution de 1,5 bars.

On obtient ainsi un lit fluidisé de granules dont la granulométrie est majoritairement comprise entre 100 et 500 µm

On procède ensuite à l'enrobage des granules en pulvérisant la fraction restante de la solution de polymère sur le lit fluidisé de granules, la durée de pulvérisation étant d'environ 2 heures, le débit de pulvérisation d'environ 15 à 20 grammes/minutes et la pression d'atomisation de la solution de 2 bars.

On obtient alors un mélange de granules enrobés.

Le mélange homogène de granules enrobés a une composition statistiquement constante.

Ainsi, il est possible de déterminer la teneur en principe actif d'une masse donnée de ce mélange homogène de granules enrobés, et, inversement, il est possible pour une quantité de principe actif souhaitée de déterminer la quantité correspondante de mélange homogène de granules enrobés.

Par exemple, pour avoir 20 mg de Lisinopril, il faudra prendre 49 mg du mélange homogène de granules enrobés. La composition de ces 47,2 mg est indiquée dans le tableau XI ci-dessous.

**Tableau XI**

| | | |
|---|---|---|
| Principe actif | Lisinopril | 20 mg |
| Agent diluant | Mannitol | 20 mg |
| Agent liant/Agent d'enrobage | Eudragit® E100 | 5,2 mg |
| Agent antistatique | Silice précipitée | 2 mg |
| | TOTAL | 47,2 mg |

### Exemple 12 : Préparation de comprimés multiparticulaires à délitement rapide contenant 20 mg de Lisinopril

On prépare des comprimés contenant 20mg de Lisinopril dont la composition résulte du tableau XII ci-après

**Tableau XII**

| **Composition d'un comprimé contenant 20 mg de Lisinopril** | | |
|---|---|---|
| Principe actif | Granules enrobés de l'exemple 11 | 47,2 mg équivalent à 20 mg de Lisinopril |
| Agent de compression | Mannitol | 120 mg |
| Agent désintégrant | Crospovidone | 12 mg |
| Agent perméabilisant | Silice précipitée | 2 mg |
| Agent édulcorant | Aspartam | 3 mg |
| Aromatisant | Arôme Menthe | 2 mg |
| Agent lubrifiant | Stéarate de magnésium | 1,8 mg |

Pour la préparation de ces comprimés on procède comme dans l'exemple 2 en utilisant les excipients et le principe actif dans les proportions relatives résultant du tableau XII ci-dessus.

Le poids des comprimés est de 188 mg.

La dureté des comprimés obtenus est de 35 N environ.

Le temps de désagrégation dans la bouche des comprimés ainsi obtenus est inférieur à 25 secondes.

L'abrasion, mesurée comme dans l'exemple 2, a été trouvée comme étant inférieure à 1 %.

Le goût désagréable du Lisinopril n'est pas détecté pendant le temps de séjour du comprimé dans la bouche, c'est à dire entre le moment de son administration et la déglutition.

### Exemple 13 : Préparation de granules enrobés de Trandolapril.

Les granules sont fabriqués selon le procédé suivant.

On prépare tout d'abord une solution de polymère en solubilisant l'Eudragit® E100 dans de l'alcool isopropylique.

On fluidise ensuite un mélange homogène de poudres constitué de 250g de Trandolapril, 750g de mannitol et 50g de silice précipitée dans un lit d'air fluidisé.

On réalise la granulation par pulvérisation sur le lit fluidisé du mélange homogène de poudres, d'une première fraction d'environ 15 à 25 % de la solution de polymère pendant environ 30 minutes à un débit de pulvérisation d'environ 20 à 25 grammes/minutes et une pression d'atomisation de la solution de 1,5 bars.

On obtient ainsi un lit fluidisé de granules dont la granulométrie est majoritairement comprise entre 100 et 500 µm

On procède ensuite à l'enrobage des granules en pulvérisant la fraction restante de la solution de polymère sur le lit fluidisé de granules, la durée de pulvérisation étant d'environ 2 heures, le débit de pulvérisation d'environ 15 à 20 grammes/minutes et la pression d'atomisation de la solution de 2 bars.

On obtient alors un mélange de granules enrobés.

Le mélange homogène de granules enrobés a une composition statistiquement constante.

Ainsi, il est possible de déterminer la teneur en principe actif d'une masse donnée de ce mélange homogène de granules enrobés, et, inversement, il est possible pour une quantité de principe actif souhaitée de déterminer la quantité correspondante de mélange homogène de granules enrobés.

Par exemple, pour avoir 4 mg de Trandolapril, il faudra prendre 18,4 mg du mélange homogène de granules enrobés. La composition de ces 18,4 mg est indiquée dans le tableau XIII ci-dessous

**Tableau XIII**

| | | |
|---|---|---|
| Principe actif | Trandolapril | 4 mg |
| Agent diluant | Mannitol | 12 mg |
| Agent liant/Agent d'enrobage | Eudragit® E100 | 1,6 mg |
| Agent antistatique | Silice précipitée | 0,8 mg |
| | TOTAL | 18,4 mg |

### Exemple 14 : Préparation de comprimés multiparticulaires à délitement rapide contenant 4 mg de Trandolapril

On prépare des comprimés contenant 4mg de Trandolapril dont la composition résulte du tableau XIV ci-après :

**Tableau XIV**

| **Composition d'un comprimé contenant 4 mg de Trandolapril** | | |
|---|---|---|
| Principe actif | Granules enrobés de l'exemple 13 | 18,4 mg équivalent à 4 mg de Trandolapril |
| Agent de compression | Mannitol | 117,6 mg |
| Agent désintégrant | Crospovidone | 16 mg |
| Agent perméabilisant | Silice précipitée | 1,5 mg |
| Agent édulcorant | Aspartam | 3 mg |
| Aromatisant | Arôme Menthe | 2 mg |
| Agent lubrifiant | Stéarate de magnésium | 1,5 mg |

Pour la préparation de ces comprimés on procède comme dans l'exemple 2 en utilisant les excipients et le principe actif dans les proportions relatives résultant du tableau XIV ci-dessus.

Le poids des comprimés est de 160 mg.

La dureté des comprimés obtenus est de 35 N environ.

Le temps de désagrégation dans la bouche des comprimés ainsi obtenus est inférieur à 15 secondes.

L'abrasion, mesurée comme dans l'exemple 2, a été trouvée comme étant inférieure à 1 %.

Le goût désagréable du Trandolapril n'est pas détecté pendant le temps de séjour du comprimé dans la bouche, c'est à dire entre le moment de son administration et la déglutition.

## Revendications

1. Granules à base d'inhibiteur de l'enzyme de conversion de l'angiotensine, de ses isomères ou de ses sels pharmaceutiquement acceptables (IEC) **caractérisés par le fait qu'**ils sont enrobés et qu'ils contiennent :
- des microcristaux d'IEC,
- un ou plusieurs agents liants choisis dans le groupe comprenant notamment les polymères cellulosiques, en particulier l'éthylcellulose, l'hydroxypropylcellulose et l'hydroxypropylméthylcellulose, les polymères acryliques, les povidones, les polyvinylalcools, et leurs mélanges,
- éventuellement un agent diluant choisi dans le groupe comprenant notamment les dérivés cellulosiques, les amidons, le lactose, les polyols, en particulier le mannitol, et un agent antistatique choisi dans le groupe comprenant notamment la silice colloïdale, la silice précipitée et le talc micronisé ou non.

2. Granules selon la revendication 1, **caractérisés par le fait que** L'IEC est choisi dans le groupe comprenant notamment le Bénazépril, le Captopril, le Cilazapril, l'Enalapril, le Sosinopril, le Lisinopril, le Périndopril, le Quinapril, le Ramipril, le Trandolapril, leurs isomères et leurs sels pharmaceutiquement acceptables.

3. Granules selon la revendication 2, **caractérisés par le fait que** l'IEC est choisi dans le groupe comprenant le Ramipril, le Captopril, l'Enalapril, le Lisinopril, le Trandolapril, leurs isomères ou leurs sels pharmaceutiquement acceptables.

4. Granules selon l'une quelconque des revendications 1 à 3, **caractérisés par le fait qu'**ils présentent une granulométrie telle qu'au moins 80% des granules ont une taille comprise entre 100 et 500µm, et moins de 15% des granules ont une taille inférieure à 100µm.

5. Granules selon l'une quelconque des revendications 1 à 4, **caractérisés par le fait que**:
- la quantité d'agent liant est d'au plus 15% en poids, de préférence d'au plus 10% en poids par rapport au poids des granules non enrobés,
- la quantité d'agent diluant est d'au plus 85% en poids, de préférence d'au plus 50% en poids par rapport au poids des granules non enrobés,
- la quantité d'agent antistatique est d'au plus 10% en poids, de préférence d'au plus 3% en poids par rapport au poids des granules non enrobés.

6. Granules selon l'une quelconque des revendications 1 à 5, **caractérisés par le fait qu'**ils sont enrobés par une composition d'enrobage comprenant un polymère d'enrobage choisi dans le groupe comprenant les polymères cellulosiques en particulier l'éthylcellulose, l'hydroxypropylcellulose et l'hydroxypropylméthylcellulose, les polymères acryliques, et leur mélange, et éventuellement un agent antistatique, des plastifiants, et des agents solubles, notamment des polyols.

7. Granules selon l'une quelconque des revendications 1 à 6, **caractérisés par le fait qu'**un même polymère constitue l'agent liant du granule non enrobé et le polymère d'enrobage.

8. Granules selon la revendication 7, **caractérisés par le fait qu'**ils comprennent:
- de 10 à 95% de microcristaux d'IEC,
- de 0 à 85% d'un agent diluant, de préférence de mannitol,
- de 0 à 10% d'un agent antistatique, de préférence de silice colloïdale,
- de 5 à 10% de polymère d'enrobage ou agent liant, de préférence d'éthylcellulose,
les pourcentages étant exprimés en poids par rapport au poids du granule enrobé.

9. Procédé de préparation de granules enrobés selon l'une quelconque des revendications 1 à 8, **caractérisé par le fait qu'**il comprend successivement les étapes:
- de mélange à sec des microcristaux d'IEC avec l'agent diluant et éventuellement un agent antistatique,
- de granulation du mélange obtenu à l'étape précédente par pulvérisation d'une solution ou suspension de l'agent liant,
- d'enrobage des granules ainsi obtenus par pulvérisation d'une suspension de la composition d'enrobage,
- de séchage des granules enrobés ainsi obtenus.

10. Procédé selon la revendication 9, **caractérisé par le fait que** les différentes étapes sont effectuées dans un dispositif à air fluidisé.

11. Procédé selon l'une des revendications 9 ou 10, **caractérisé par le fait que** le débit de pulvérisation de la solution ou suspension d'agent liant lors de l'étape de granulation est plus élevé que le débit de pulvérisation de la suspension de la composition d'enrobage lors de l'étape d'enrobage et la pression d'atomisation est moins élevée lors de l'étape de granulation que lors de l'étape d'enrobage.

12. Comprimé à délitement rapide du genre de ceux qui sont destinés à se désagréger dans la bouche au contact de la salive en moins de 60 secondes, de préférence en moins de 40 secondes, en formant une suspension aisée à avaler, **caractérisé par le fait qu'**il est à base de granules enrobés selon l'une quelconque des revendications 1 à 8 ou tels que préparés selon le procédé des revendications 9 à 11 et d'un mélange d'excipients comprenant au moins un agent de désintégration, un agent soluble diluant, un agent lubrifiant et éventuellement un agent gonflant, un agent perméabilisant, des édulcorants et des arômes.

13. Comprimé selon la revendication 12, **caractérisé par le fait que** l'agent de désintégration est choisi dans le groupe comprenant notamment la croscarmellose, la crospovidone et leurs mélanges.

14. Comprimé selon l'une des revendications 12 ou 13, **caractérisé par le fait que** l'agent soluble a des propriétés liantes et est constitué par un polyol de moins de 13 atomes de carbone se présentant soit sous la forme du produit directement compressible dont le diamètre moyen des particules est de 100 à 500µm, soit sous la forme d'une poudre dont le diamètre moyen des particules est inférieur à 100µm, ce polyol étant de préférence choisi dans le groupe comprenant le mannitol, le xylitol, le sorbitol et le maltitol, étant entendu que le sorbitol ne peut être utilisé seul et que, dans le cas où l'agent soluble diluant à propriétés liantes est unique, il est utilisé sous la forme du produit directement compressible alors que, dans le cas où il y a au moins deux agents solubles diluants à propriétés liantes, l'un est présent sous la forme directement compressible et l'autre sous la forme de poudre, le polyol pouvant alors être le même, les proportions de polyol directement compressible et de polyol poudre étant de 99/1 à 20/80, de préférence de 80/20 à 20/80, plus préférentiellement encore de 80/20 à 50/50.

15. Comprimé selon l'une quelconque des revendications 12 à 14, **caractérisé par le fait que** la proportion de mélange d'excipients par rapport aux granules enrobés d'IEC est de 0,4 à 10, de préférence de 1 à 10, et plus préférentiellement encore de 1 à 4 parties en poids.

16. Comprimé selon l'une quelconque des revendications 12 à 15, **caractérisé par le fait que** la proportion d'agent de désintégration par rapport à la masse de comprimé est de 1 à 15%"et de préférence de 2 à 7% en poids et la proportion d'agent soluble par rapport à la masse du comprimé est de 30 à 90% et de préférence de 40 à 70% en poids.

17. Comprimé selon l'une quelconque des revendications 12 à 16, **caractérisé par le fait que** l'agent perméabilisant est choisi dans le groupe comprenant notamment les silices présentant une grande affinité pour les solvants aqueux , telles que la silice précipitée, les maltodextrines, les β-cyclodextrines et leurs mélanges.

18. Comprimé selon l'une quelconque des revendications 12 à 17, **caractérisé par le fait que** le lubrifiant est choisi dans le groupe comprenant notamment le stéarate de magnésium, le stéarylfumarate de sodium, l'acide stéarique, le polyoxyéthylèneglycol micronisé et leurs mélanges.

19. Comprimé selon l'une quelconque des revendications 12 à 18, **caractérisé par le fait que** l'édulcorant est choisi dans le groupe comprenant notamment l'aspartam, l'acesulfame de potassium, le saccharinate de potassium, la néohespéridine dihydrochalcone, le sucralose, le monoammonium glycyrrhizinate, et leurs mélanges.

20. Comprimé selon l'une quelconque des revendications 12 à 19, **caractérisé par le fait que** l'agent lubrifiant est sous forme de poudre et se trouve réparti pour au moins sa majeure partie à la surface du comprimé.

## Claims

1. Granules based on angiotensin converting enzyme inhibitor, its isomers or its pharmaceutically acceptable salts (IEC), **characterized in that** they are coated and **in that** they contain:
- microcrystals of IEC,
- one or more binders selected from the group comprising especially cellulosic polymers, particularly ethyl cellulose, hydroxypropyl cellulose and hydroxypropyl methyl cellulose, acrylic polymers, povidones, polyvinyl alcohols and mixtures thereof,
- optionally a diluent selected from the group comprising especially cellulosic derivatives, starches, lactose and polyols, particularly mannitol, and an antistatic agent selected from the group comprising especially colloidal silica, precipitated silica and micronized or non-micronized talcum.

2. Granules according to Claim 1, **characterized in that** the IEC is selected from the group comprising especially benazepril, captopril, cilazapril, enalapril, sosinopril, lisinopril, perindopril, quinapril, ramipril, trandolapril, their isomers and their pharmaceutically acceptable salts.

3. Granules according to Claim 2, **characterized in that** the IEC is selected from the group comprising ramipril, captopril, enalapril, lisinopril, trandolapril, their isomers and their pharmaceutically acceptable salts.

4. Granules according to any one of Claims 1 to 3, **characterized in that** they have a particle size distribution such that at least 80% of the granules have a size of between 100 and 500 µm, and less than 15% of the granules have a size below 100 µm.

5. Granules according to any one of Claims 1 to 4, **characterized in that**:
- the amount of binder is at most 15% by weight, preferably at most 10% by weight, based on the weight of the uncoated granules,
- the amount of diluent is at most 85% by weight, preferably at most 50% by weight, based on the weight of the uncoated granules, and
- the amount of antistatic agent is at most 10% by weight, preferably at most 3% by weight, based on the weight of the uncoated granules.

6. Granules according to any one of Claims 1 to 5, **characterized in that** they are coated with a coating composition comprising a coating polymer selected from the group comprising cellulosic polymers, particularly ethyl cellulose, hydroxypropyl cellulose and hydroxypropyl methyl cellulose, acrylic polymers and mixtures thereof, and optionally an antistatic agent, plasticizers and soluble agents, especially polyols.

7. Granules according to any one of Claims 1 to 6, **characterized in that** one and the same polymer constitutes the binder of the uncoated granule and the coating polymer.

8. Granules according to Claim 7, **characterized in that** they comprise:
- from 10 to 95% of microcrystals of IEC,
- from 0 to 85% of a diluent, preferably mannitol,
- from 0 to 10% of an antistatic agent, preferably colloidal silica, and
- from 5 to 10% of coating polymer or binder, preferably ethyl cellulose,
the percentages being expressed by weight, based on the weight of the coated granule.

9. Process for the preparation of coated granules according to any one of Claims 1 to 8, **characterized in that** it comprises the following successive steps:
- dry mixing of the microcrystals of IEC with the diluent and optionally an antistatic agent,
- granulation of the mixture obtained in the previous step by spraying with a solution or suspension of the binder,
- coating of the granules obtained by spraying with a suspension of the coating composition, and
- drying of the coated granules obtained.

10. Process according to Claim 9, **characterized in that** the different steps are carried out in a fluidized air device.

11. Process according to Claim 9 or 10, **characterized in that** the spraying rate of the solution or suspension of binder in the granulation step is higher than the spraying rate of the suspension of coating composition in the coating step, and the atomization pressure is lower in the granulation step than in the coating step.

12. Rapidly disintegrating tablet of the kind intended to disaggregate in the mouth on contact with the saliva in less than 60 seconds, preferably in less than 40 seconds, to form a suspension that is easy to swallow, **characterized in that** it is based on coated granules according to any one of Claims 1 to 8 or coated granules as prepared by the process of Claims 9 to 11 and a mixture of excipients comprising at least one disintegrating agent, a soluble diluent, a lubricant and optionally a swelling agent, a permeabilizing agent, sweeteners and flavourings.

13. Tablet according to Claim 12, **characterized in that** the disintegrating agent is selected from the group comprising especially croscarmellose, crospovidone and mixtures thereof.

14. Tablet according to Claim 12 or 13, **characterized in that** the soluble agent has binding properties and consists of a polyol having fewer than 13 carbon atoms which takes the form either of the directly compressible product having a mean particle diameter of 100 to 500 µm, or of a powder having a mean particle diameter below 100 µm, this polyol preferably being selected from the group comprising mannitol, xylitol, sorbitol and maltitol, it being understood that sorbitol cannot be used on its own and that, in the case where there is only one soluble diluent with binding properties, it is used in the form of the directly compressible product, whereas in the case where there are at least two soluble diluents with binding properties, one is present in directly compressible form and the other in powder form, it then being possible for the polyol to be the same, the proportions of directly compressible polyol and powdered polyol being from 99/1 to 20/80, preferably from 80/20 to 20/80 and particularly preferably from 80/20 to 50/50.

15. Tablet according to any one of Claims 12 to 14, **characterized in that** the proportion of excipient mixture relative to coated granules of IEC is from 0.4 to 10, preferably from 1 to 10 and particularly preferably from 1 to 4 parts by weight.

16. Tablet according to any one of Claims 12 to 15, **characterized in that** the proportion of disintegrating agent relative to the weight of the tablet is from 1 to 15% by weight, preferably from 2 to 7% by weight, and the proportion of soluble agent relative to the weight of the tablet is from 30 to 90% by weight, preferably from 40 to 70% by weight.

17. Tablet according to any one of Claims 12 to 16, **characterized in that** the permeabilizing agent is selected from the group comprising especially silicas having a high affinity for aqueous solvents, such as precipitated silica, maltodextrins, β-cyclodextrins and mixtures thereof.

18. Tablet according to any one of Claims 12 to 17, **characterized in that** the lubricant is selected from the group comprising especially magnesium stearate, sodium stearylfumarate, stearic acid, micronized polyoxyethylene glycol and mixtures thereof.

19. Tablet according to any one of Claims 12 to 18, **characterized in that** the sweetener is selected from the group comprising especially aspartame, acesulfame potassium, potassium saccharinate, neohesperidine dihydrochalcone, sucralose, monoammonium glycyrrhizinate and mixtures thereof.

20. Tablet according to any one of Claims 12 to 19, **characterized in that** the lubricant is in powder form, at least the major part of it being distributed over the surface of the tablet.

## Patentansprüche

1. Körnchen auf Basis des Inhibitors von Angiotensin-umsetzendem Enzym (IEC), seiner Isomere oder seiner pharmazeutisch akzeptablen Salze, **dadurch gekennzeichnet, dass** sie umhüllt sind und dass sie enthalten:
- IEC-Mikrokristalle,
- eines oder mehrere Bindemittel, ausgewählt aus der Gruppe, umfassend insbesondere Cellulosepolymere, insbesondere Ethylcellulose, Hydroxypropylcellulose, und Hydroxypropylmethylcellulose, Acrylpolymere, Povidone, Polyvinylalkohole und deren Gemische,
- gegebenenfalls ein Streckmittel, ausgewählt aus der Gruppe, umfassend insbesondere Cellulosederivate, Stärken, Lactose, Polyole, insbesondere Mannitol, und ein Antistatikmittel, ausgewählt aus der Gruppe, umfassend insbesondere kolloidale Kieselerde, präzipitierte Kieselerde und mikronisierten oder nicht-mikronisierten Talk.

2. Körnchen nach Anspruch 1, **dadurch gekennzeichnet, dass** der IEC ausgewählt ist aus der Gruppe, umfassend insbesondere Benazepril, Captopril, Cilazapril, Enalapril, Sosinopril, Lisinopril, Perindopril, Quinapril, Ramipril, Trandolapril, ihren Isomeren und ihren pharmazeutisch akzeptablen Salzen.

3. Körnchen nach Anspruch 1, **dadurch gekennzeichnet, dass** der IEC ausgewählt ist aus der Gruppe, umfassend insbesondere Ramipril, Captopril, Enalapril, Lisinopril, Trandolapril, ihren Isomeren oder ihren pharmazeutisch akzeptablen Salzen.

4. Körnchen nach einem der Ansprüche 1 bis 3, **dadurch gekennzeichnet, dass** sie eine derartige Korngrößenverteilung haben, so dass mindestens 80% der Körnchen eine Größe zwischen 100 und 500 µm, und weniger als 15% der Körnchen eine Größe kleiner 100 µm haben.

5. Körnchen nach einem der Ansprüche 1 bis 4, **dadurch gekennzeichnet, dass**:
- die Menge an Bindemittel höchstens 15 Gew.-%, bevorzugt höchstens 10 Gew.-%, bezogen auf das Gewicht der nicht umhüllten Körnchen, beträgt,
- die Menge an Streckmittel höchstens 85 Gew.-%, bevorzugt höchstens 50 Gew.-%, bezogen auf das Gewicht der nicht umhüllten Körnchen, beträgt,
- die Menge an Antistatikmittel höchstens 10 Gew.-%, bevorzugt höchstens 3 Gew.-%, bezogen auf das Gewicht der nicht umhüllten Körnchen, beträgt.

6. Körnchen nach einem der Ansprüche 1 bis 5, **dadurch gekennzeichnet, dass** sie umhüllt sind mit einer Umhüllungszusammensetzung, umfassend ein Umhüllungspolymer, ausgewählt aus der Gruppe, umfassend Cellulosepolymere, insbesondere Ethylcellulose, Hydroxypropylcellulose, und Hydroxypropylmethylcellulose, Acrylpolymere und deren Gemische, und gegebenenfalls ein Antistatikmittel, Plastifizierungsmittel und lösliche Mittel, insbesondere Polyole.

7. Körnchen nach einem der Ansprüche 1 bis 6, **dadurch gekennzeichnet, dass** dass das gleiche Polymer das Bindemittel des nicht umhüllten Körnchens und das Umhüllungspolymer bildet.

8. Körnchen nach Anspruch 7, **dadurch gekennzeichnet, dass** sie umfassen:
- 10 bis 95 % IEC-Mikrokristalle,
- 0 bis 85 % eines Streckmittels, bevorzugt Mannitol,
- 0 bis 10 % eines Antistatikmittels, bevorzugt kolloidale Kieselerde,
- 5 bis 10 % Umhüllungspolymer oder Bindemittel, bevorzugt Ethylcellulose,
wobei die Prozentangaben als Gewicht, bezogen auf das Gewicht der umhüllten Körnchen ausgedrückt sind.

9. Verfahren zur Herstellung von umhüllten Körnchen nach einem der Ansprüche 1 bis 8, **dadurch gekennzeichnet, dass** es nacheinander die Schritte umfasst:
- Trockenmischen von IEC-Mikrokristallen mit Streckmittel und gegebenenfalls einem Antistatikmittel,
- Granulieren des im vorangehenden Schritts erhaltenen Gemisches durch Zerstäuben einer Bindemittellösung oder -suspension,
- Umhüllen der derart erhaltenen Körnchen durch Zerstäuben einer Suspension der Umhüllungszusammensetzung,
- Trocknen der derart erhaltenen umhüllten Körnchen.

10. Verfahren nach Anspruch 9, **dadurch gekennzeichnet, dass** die verschiedenen Schritte in einer Fließbettvorrichtung durchgeführt werden.

11. Verfahren nach einem der Ansprüche 9 oder 10, **dadurch gekennzeichnet, dass** der Durchsatz beim Zerstäuben der Bindemittellösung oder -suspension beim Granulierungsschritt höher ist als der Durchsatz beim Zerstäuben der Suspension der Umhüllungszusammensetzung beim Umhüllungsschritt, und dass der Zerstäubungsdruck beim Granulierungsschritt niedriger ist als beim Umhüllungsschritt.

12. Schnell zerfallende Tablette des Typs, die dazu vorgesehen sind, im Mund bei Kontakt mit Speichel in weniger als 60 Sekunden, bevorzugt in weniger als 40 Sekunden zu zerfallen, unter Bildung einer leicht zu schluckenden Suspension, **dadurch gekennzeichnet, dass** sie basiert auf umhüllten Körnchen nach einem der Ansprüche 1 bis 8, oder Körnchen, hergestellt durch das Verfahren der Ansprüche 9 bis 11, und einem Gemisch von Hilfsstoffen, umfassend mindestens ein Zerfallmittel, ein lösliches Streckmittel, ein Gleitmittel und gegebenenfalls ein Blähmittel, ein Permeabilisierungsmittel, Süßstoffe und Geschmacksstoffe.

13. Tablette nach Anspruch 12, **dadurch gekennzeichnet, dass** das Zerfallmittel ausgewählt ist aus der Gruppe, umfassend insbesondere Croscarmellose, Crospovidon, und deren Gemische.

14. Tablette nach einem der Ansprüche 12 oder 13, **dadurch gekennzeichnet, dass** das lösliche Mittel Bindemitteleigenschaften aufweist und durch ein Polyol mit mindestens 13 Kohlenstoffen gebildet ist, das entweder in Form des direkt verpressbaren Produkts mit einem mittleren Partikeldurchmesser von 100 bis 500 µm oder in Form eines Pulvers mit einem mittleren Partikeldurchmesser kleiner 100 µm vorliegt, wobei dieses Polyol bevorzugt ausgewählt ist aus der Gruppe, umfassend Mannitol, Xylitol, Sorbitol und Maltitol, wobei verstanden wird, dass Sorbitol nicht alleine verwendet werden kann, und dass, wenn nur ein lösliches Streckmittel mit Bindemitteleigenschaften vorhanden ist, dieses in Form des direkt verpressbaren Produkts verwendet wird, während wenn mindestens zwei lösliche Streckmittel mit Bindemitteleigenschaften vorhanden sind, das eine in der direkt verpressbaren Form und das andere in Pulverform vorhanden ist, wobei das Polyol das gleiche sein kann, und die Verhältnisse von direkt verpressbarem Polyol zu pulverförmigem Polyol 99/1 bis 20/80, bevorzugt 80/20 bis 20/80, noch stärker bevorzugt 80/20 bis 50/50 betragen.

15. Tablette nach einem der Ansprüche 12 bis 14, **dadurch gekennzeichnet, dass** der Anteil des Gemisches von Hilfsstoffen, bezogen auf mit IEC umhüllte Körnchen, 0,4 bis 10, bevorzugt 1 bis 10, und noch stärker bevorzugt 1 bis 4 Gewichtsteile beträgt.

16. Tablette nach einem der Ansprüche 12 bis 15, **dadurch gekennzeichnet, dass** der Anteil von Zerfallmittel, bezogen auf die Masse der Tablette, 1 bis 15 Gew.-% und bevorzugt 2 bis 7 Gew.-% beträgt, und dass der Anteil von löslichem Mittel, bezogen auf die Masse der Tablette, 30 bis 90 Gew.-% und bevorzugt 40 bis 70 Gew.-% beträgt.

17. Tablette nach einem der Ansprüche 12 bis 16, **dadurch gekennzeichnet, dass** das Permeabilisierungsmittel ausgewählt ist aus der Gruppe, umfassend insbesondere die Kieselerden, welche eine hohe Affinität für wässrige Lösungsmittel aufweisen, wie etwa präzipitierte Kieselerde, Maltodextrine, β-Cyclodextrine, und deren Gemische.

18. Tablette nach einem der Ansprüche 12 bis 17, **dadurch gekennzeichnet, dass** das Gleitmittel ausgewählt ist aus der Gruppe, umfassend insbesondere Magnesiumstearat, Natriumstearylfumarat, Stearinsäure, mikronisiertes Polyoxyethylenglycol, und deren Gemische.

19. Tablette nach einem der Ansprüche 12 bis 18, **dadurch gekennzeichnet, dass** der Süßstoff ausgewählt ist aus der Gruppe, umfassend insbesondere Aspartam, Kaliumacesulfam, Kaliumsaccharinat, Neohesperidindihydrochalcon, Sucralose, Monoammoniumglycyrrhizinat, und deren Gemischen.

20. Tablette nach einem der Ansprüche 12 bis 19, **dadurch gekennzeichnet, dass** das Gleitmittel in Form von Pulver vorliegt und sich mindestens der Großteil von ihm auf der Oberfläche der Tablette verteilt befindet.
